# EUROPEAN PATENT SPECIFICATION

(11) **EP 4 299 719 B1**
(45) Date of publication and mention of the grant of the patent: **31.12.2025**
(21) Application number: 23181590.3
(22) Date of filing: 27.06.2023
(51) Int. Cl.: C12N 5/00, C12N 5/071, C12N 5/077, A61L 27/38, A61L 27/44, A61L 27/60, B33Y 70/00, B33Y 80/00, G09B 23/30

(54) **A COMPLEX IN VITRO MODEL OF HUMAN SKIN, A PROCESS FOR PREPARATION AND USE THEREOF**
KOMPLEXES IN-VITRO-MODELL DER MENSCHLICHEN HAUT, VERFAHREN ZUR HERSTELLUNG UND VERWENDUNG DAVON
MODÈLE COMPLEXE IN VITRO DE PEAU HUMAINE, PROCÉDÉ DE PRÉPARATION ET UTILISATION DE CELUI-CI

(30) Priority: 28.06.2022 LU 502391
(43) Date of publication of application: 03.01.2024
(73) Proprietor: Univerza v Mariboru, 2000 Maribor (SI)
(72) Inventor: Maver, Tina, 2000 Maribor (SI); Maver, Uros, 2000 Maribor (SI); Zidaric, Tanja, 2000 Maribor (SI); Gradisnik, Lidija, 2000 Maribor (SI)
(74) Representative: Patentni Biro AF d.o.o.

(56) References cited:
- WO-A1-2019/113442
- T�NIA BALTAZAR ET AL: "Three Dimensional Bioprinting of a Vascularized and Perfusable Skin Graft Using Human Keratinocytes, Fibroblasts, Pericytes, and Endothelial Cells", TISSUE ENGINEERING PART A, vol. 26, no. 5-6, 3 December 2019 (2019-12-03), US, pages 227 - 238, XP055717471, ISSN: 1937-3341, DOI: 10.1089/ten.tea.2019.0201
- BALTAZAR TANIA ET AL: "bioprinting of an implantable xeno-free vascularized human skin graft", vol. 8, no. 1, 21 April 2022 (2022-04-21), XP093022629, ISSN: 2380-6761, Retrieved from the Internet <URL:https://onlinelibrary.wiley.com/doi/full-xml/10.1002/btm2.10324> DOI: 10.1002/btm2.10324
- CAROLINA MOTTER CATARINO: "Development of 3D bioprinted reconstructed skin models using native and non-native bioinks", 1 August 2018 (2018-08-01), XP055717474, Retrieved from the Internet <URL:http://metrologia.org.br/eventos/pan2018_palestras/SECTION%20I_4_Carolina%20Motter%20Catarino.pdf> [retrieved on 20200723]
- SALAMEH SACHA ET AL: "A perfusable vascularized full-thickness skin model for potential topical and systemic applications", BIOFABRICATION, vol. 13, no. 3, 1 July 2021 (2021-07-01), UK, pages 035042, XP093022700, ISSN: 1758-5082, DOI: 10.1088/1758-5090/abfca8
- RIMAL RAHUL ET AL: "Dynamic flow enables long-term maintenance of 3-D vascularized human skin models", APPLIED MATERIALS, vol. 25, 1 December 2021 (2021-12-01), NL, pages 101213, XP093022703, ISSN: 2352-9407, DOI: 10.1016/j.apmt.2021.101213
- ZOIO PATR�CIA ET AL: "Skin-on-a-Chip Technology: Microengineering Physiologically Relevant In Vitro Skin Models", vol. 14, no. 3, 21 March 2022 (2022-03-21), pages 682, XP093022643, Retrieved from the Internet <URL:https://www.ncbi.nlm.nih.gov/pmc/articles/PMC8955316/pdf/pharmaceutics-14-00682.pdf> DOI: 10.3390/pharmaceutics14030682
- BEHESHTIZADEH NIMA ET AL: "A review of 3D bio-printing for bone and skin tissue engineering: a commercial approach", JOURNAL OF MATERIAL SCIENCE, KLUWER ACADEMIC PUBLISHERS, DORDRECHT, vol. 55, no. 9, 4 December 2019 (2019-12-04), pages 3729 - 3749, XP036975490, ISSN: 0022-2461, [retrieved on 20191204], DOI: 10.1007/S10853-019-04259-0

## Description

### Field of the invention

The present invention belongs to the field of medicine and tissue engineering, more precisely to the field of preparation of *in vitro* models of human organs. The invention also belongs to the field of testing materials, chemical agents, pharmaceutical preparations and similar, as well as to the field of diagnostics and monitoring of diseases. The invention relates to a complex *in vitro* model of human skin, preferably with a layer of bone tissue, and a process for its production. In addition, the invention also relates to the use of the prepared *in vitro* model, which enables non-invasive measurements of blood glucose levels.

### Background of the invention and the technical problem

Biomedical research is closely related to clinical trials, which represent a huge financial burden for investors. *In vitro* models are an important part of medicine and biology because they provide insight into how cells, tissues, and organs work. These models, which provide representative testing of newly developed drugs, cosmetics, and medical supplies are therefore extremely important in reducing the number and/or scope of *in vivo* clinical trials. *In vitro* models of various organs have already been developed. Among them, skin models are one of the most well-known, as various industries, such as pharmaceutical, cosmetic, and medical accessory (sensor) industries, have common interests. A major problem with known skin models is their low complexity, as they include only a few cell types or structures that are important for mimicking real skin *in vivo.* For example, some models consist only of dermis or dermis and epidermis, leaving out several other important components such as the nervous system, vascular networks, adipose tissue and similar.

For example, patent EP0197090B1 describes a skin equivalent consisting only of dermis, which is made of collagen, fibroblasts, and a hydrated collagen network. Keratinocytes representing the epidermis are not included in the model, but the *in vitro* dermis described should allow for the growth of keratinocytes on its surface. A similar collagen network functionalized with fibroblasts is also described in patent EP0418035B1 and patent application WO2001092477A2, while patent application WO9510600 describes an epidermis model suitable for testing cosmetic products.

The enhancement of a skin layer, the dermis or epidermis, with an additional layer has been described in several documents: EP0285474B1, EP0020753B1, US5755814, WO01/092477, US5755814A, US6846675B2, WO2012133629A1, using various materials as base material, including collagen, various hydrogels and similar.

Because the cells in the *in vitro* model are taken from their natural environment, the models described above cannot ideally mimic a real tissue or organ. Therefore, it must be kept in mind that the observed effects or events do not represent a complete and/or accurate picture of all the effects of a particular tested material, chemical, or pharmaceutical product on the entire system, which is not ideal in practice. Developing a complex *in vitro* skin model would allow reliable results to be obtained quickly, while avoiding *in vivo* animal testing and limiting clinical trials to what is necessary. The time from an idea to a final product for application on the skin would thus be significantly shorter. More importantly, the resulting reduction in testing costs could lead to faster time-to-market for new products and their clinical application directly benefiting patients.

Instead of complex organ models, some researchers are striving to use microchips, which are microfluidic devices containing cells of the desired organs. One such solution is described in document KR2018004040 and in the article »Skin-on-a-chip model simulating inflammation, oedema and drug-based treatment« (Scientific Reports 6, 37471; 2016). Both documents refer to the preparation of a microchip of skin tissue and blood vessels on which epidermis, dermis, and endothelial layers were seeded with human cells. Due to the limited dimensions of the chips, it is impossible to ensure a reliable and meaningful simulation of events in the skin due to the lack of suitable mechanical, structural, physiological and other parameters. Therefore, these solutions cannot replace the need for *in vitro* models.

Thus there is a need for the development of *in vitro* models of organs that mimic the structure and function of organs as closely as possible to provide test systems with high reliability and high similarity to the actual condition in a human or animal body. Therefore, the technical problem addressed by the present invention is the development of a complex *in vitro* skin model that mimics and simultaneously enables all major components of human skin and at the same time enables:
- *in vitro* testing of transdermal pharmaceutical and cosmetic preparations,
- the study of the passage of active ingredients from the vascular system into the adjacent tissues, and
- skin measurements of parameters such as glucose and cholesterol that are more comparable to *in vivo* tests.

### State of the art

Different S solutions of the *in vitro* skin model, which are less complex and therefore cannot sufficiently mimic the real physiological conditions in the skin.

Patent application WO2007090575A1 refers to a skin model that includes epidermis, dermis and dendritic cells, which should allow sensory properties of the model. Although the model has been enhanced by incorporating cells that enable sensory functions, this model still lacks blood vessels, subcutaneous tissue, and bone.

The article »Bioengineering Dermo-Epidermal Skin Grafts with Blood and Lymphatic Capillaries« (Sci Transl Med, 2014, 6(221):221ra14; doi: 10.1126/scitranslmed.3006894) describes the use of a hydrogel with added human dermal endothelial cells, keratinocytes and fibroblasts. This solution is not intended for *in vitro* testing, but is suitable for use as a skin implant in large wounds and therefore lacks other important skin components such as blood vessels and subcutaneous tissue.

To perform epidermal irritation tests, the authors of the article »Microfluidics-based skin irritation test using in vitro 3D angiogenesis platform« (APL Bioengineering 3, 036101 (2019); https://doi.org/10.1063/1.5093975) described the development of a microfluidic device with channels in which keratinocytes and endothelial cells were used to perform *in vitro* epidermal irritation tests. This model also suffers from its low complexity, as it lacks the dermis, subcutaneous tissue, and vasculature, making it suitable only for testing on the epidermis.

Another further upgrade of *in vitro* models comprises subcutaneous tissue.

Patent application JP2012235921A describes a 3D skin model with connected dermo-epidermal part and subcutaneous tissue. Separate preparation of the dermo-epidermal part (using the traditional method of 3D organotypic cell cultures on a collagen gel) and the subcutaneous tissue. For the latter, preadipocytes were used in combination with a collagen gel and cultivated separately until the required differentiation and proliferation were achieved. This model was prepared by a different method than that of the present invention and also does not include a simulation of the vasculature. Therefore, this model is not suitable for testing various physiological parameters.

The article »3D biomaterial matrix to support long term, full thickness, immunocompetent human skin equivalents with nervous system components« (Biomaterials, 2019, 198:194-203; doi: 10.1016/j.biomaterials.2018.04.044.) describes the use of neonatal keratinocytes and fibroblasts and human adipocytes. Neuron cells were also added. A gel of collagen and collagen with silk was used. Since this model does not have a vascular system, it is not possible to use it to test various non-invasive sensors and release agents from dermal/transdermal preparations.

Similarly, in the article »Improvement of a three-layered in vitro skin model for topical application of irritating substances« (Frontiers in Bioengineering 2020, 8: 388; doi: 10.3389/fbioe.2020.00388), a model with three layers (epidermis, dermis, and subcutaneous tissue) is presented in which the individual layers are prepared with collagen with isolated human cells (keratinocytes, fibroblasts and adipocytes). This model is made in a different way than the present invention and it does not mimic the morphology of real skin. In addition, this model lacks a simulation of the vascular tissue.

Ensuring flow through the skin model is essential for testing physiological functions and thus for testing new drugs or sensors. This problem has been partially addressed. Patent application CN104490489A describes 3D printed blood vessels using human neonatal fibroblasts and epithelial progenitor cells (isolated from peripheral blood) in combination with the following polymers: pluronic F127, poli(N-isopropylacrylamide), methylcellulose and sodium alginate. Once the polymer matrix is removed, only the 3D printed blood vessels remain. However, these vessels are not part of a skin system and therefore cannot be transferred and/or inserted into existing skin models.

Patent application CN12885213A discloses a three-layer 3D plastic skin model, a simulation of blood vessels and skin appendages (hair follicles, glands). The model was built on the basis of individual artificial skin layers and their bonding into a whole. This solution uses plastic materials with questionable biocompatibility, and no cells were used. Therefore, it is unlikely that this model can simulate similar body conditions as comparable *in vitro* measurements.

Patent application US2014228970A1 describes 3D printing of tubular structures using an inkjet method, where HMVEC and fibrin were used as a bioink for printing microvascular structures. Simultaneous formation of fibrin channels and deposition of HMVEC-fibrin bioink in fibrin channels within the fibrinogen substrate were described, with the printed cells aligned within the fibrin channels. This solution does not allow the formation of a skin model that comprises all layers of epidermis, dermis, and subcutaneous tissue.

The article »Three Dimensional Bioprinting of a Vascularized and Perfusable Skin Graft Using Human Keratinocytes, Fibroblasts, Pericytes, and Endothelial Cells« (Tissue Engineering Part A, 2020) describes a skin model comprising epidermis and dermis with a vascular system. Collagen I from rat tails was used as the support material, while human endothelial cells, pericytes from human placenta, and human keratinocytes were also used. This model is not fully human, as it also lacks subcutaneous tissue and vascular tissue, which are essential for *in vitro* testing.

The article »Direct 3D cell-printing of human skin with functional transwell system« (Biofabrication, 2017, 9(2):025034; doi: 10.1088/1758-5090/aa71c8) describes the preparation of a skin model comprising dermis with fibroblasts and a polycaprolactone (PCL) flow system. No subcutaneous tissue was used, which affects the mechanical properties and transfer of substances through the skin layers. PCL was used for printing vascular tissue. However, it is not possible to mimic the physiological properties of vascular tissue using PCT, so dermal and transdermal preparations cannot be tested.

Some progress has been made in the field of 3D printing of skin models and thus simulating the mechanical properties of the skin.

Patent application CN109385393A discloses a 3D printed skin model with all three layers (epidermis, dermis, and subcutaneous tissue) with a support structure. Dermis and subcutaneous tissue were printed in multiple layers, forming a matrix of natural and synthetic polymers/hydrogels with bioactive factors and cells. Preadipocytes (adipose-derived stem cells, AdSCs) were used in combination with two natural polymers for creation of subcutaneous tissue. For the dermis, fibroblasts and vascular endothelial cells were combined in a ratio from 2:1 to 5:1, while the dermis matrix was based on natural protein, preferably fibrinogen, and a polysaccharide. The epidermis was prepared directly on the dermis layer and comprises two layers, the first layer in contact with the dermis being the layer containing cells and the other layer comprises growth factors in deionized water. The disadvantage of this solution is the use of preadipocytes, since the environment in which they are printed may affect their maturation, leading to proliferation into other cells rather than adipocytes. In addition, the model lacks a vascular system, limiting its functionality and representativeness for skin.

Similarly, document SE1950711A1 describes a 3-layer printed skin model. At least one type of bioink was used in combination with at least one bioactive substance (e.g., growth factors), at least one cell source (primary or stem cells), and at least one additional cell type. This model also does not comprise a vascular system.

Closest to the present invention is the skin model described in patent application CA2864665A1. Therein, the fabrication of artificial subcutaneous tissue using inkjet 3D printing with fibrin channels and endothelial cells to simulate of blood vessels in subcutaneous tissue is described. Microvascular structures were prepared using HMVEC cells and fibrin. CD31+ cells from a lipoaspirate served as the cell source. The basis for the dermis was a combination of arbitrary cells, fibrinogen, and collagen matrix. This model has all the structures, but it does not describe the actual functionality of the vascular system that could provide flow along the simulated blood vessels. Therefore, it is questionable whether the model allows *in vitro* tests that could simulate the real events in and on the skin.

A major shortcoming in the field of skin tissue engineering today is the lack of functional flow-through vessels that could make a significant contribution to the applicability of *in vitro* skin models. Although numerous research groups are working on this topic, efficient vascularization has not yet been developed. Separately, i.e., not within an *in vitro* skin model, some solutions exist to simulate blood flow and/or the blood system, but these solutions depend on the operation of individual pumping units or compulsory hydraulic pumps. One of these systems available on the market for simulating the endothelial vascular system is the »Ibidi Pump System« developed by Ibidi GmHZ (Germany). A disadvantage of this system is its stationary operation (no possibility of relocation), its high price, and problems with its use in skin models. Therefore, this system is not suitable for the use of sensor for in *vitro* testing. In addition, there is no simulation of bone, which may be crucial for testing and validation of non-invasive sensors.

Zidaric et al. (Nanomaterials 2020, 10, 733; doi:10.3390/nano10040733) published progress in developing a of hydrogel bioink for printing dermis needed for wound healing, tissue engineering, and drug testing. A combination of nanofibrillar cellulose (NFC) with alginate (ALG), carboxymethylcellulose (CMC), and human skin fibroblasts (hSF) *in situ* was used to produce the bioink for dermis 3D printing. The results show that the bioink prepared with hSF is printable and improves proliferation. However, the article is silent on the 3D printing of whole skin models, which could be suitable for testing various preparations, drugs and materials.

Milojevic et al. (J. Vis. Exp., 2019, 151: e59951, doi:10.3791/59951) have disclosed a method for 3D core/shell bioprinting based on the use of a coaxial nozzle. In this process, 3D hydrogel structures with built-in channels are formed by controlled cross-connections that allow the formation of hollow filaments that maintain perfusion during cell cultivation and are required for vascular and other tubular tissue structures. Although this process has been disclosed, the entire skin model suitable for test applications has not been described.

Maver U. (Applied Surface Science, 2019. 489: p. 485-493) et al., and Maver T. (Journal of Sol-Gel Science and Technology, 2016. 79(3): p. 475-486) et al. described the use of different materials for the purpose of wound healing. However, this is quite different from *in vitro* skin models. The aforementioned articles investigated the potential of thin films of alginate and carboxymethylcellulose with growth factors as formulations for 3D printed materials for wound treatment or healing. These thin films were prepared on silicon wafers using spin-coating technique. 1% ALG, 1% CMC and a mixture of both in a ratio of 1:1 (ALG /CMC) were used to prepare the solutions for the production of the thin films. The growth factors EGF, bFGF and PDGF were added at concentrations comparable to their concentrations in human plasma. The results showed that EGF is most efficient for epidermis proliferation, while FGF is most efficient for fibroblast proliferation.

On the other hand, Maver T. et al., prepared a two-layer coating (dressing) for the treatment of wounds with two substances - diclofenac and lidocaine. The coating was prepared using a combination of CMC Aquacel^{®} impregnated with diclofenac for long-term pain relief in patients with wounds. Electrospinning of the biocompatible polymers CMC and polyethylene oxide (PEO) with the local anaesthetic lidocaine was performed *in situ* to provide immediate pain relief, caused by replacement of wound dressing. The CMC/PEO electrospun nanofibers have similar properties to the extracellular matrix, an important component of the skin, which positively influence the proliferation of skin cells. Thus, the described combination improves wound healing in two ways: by relieving pain and by accelerating proliferation due to ECM simulation.

### Description of the solution to the technical problem

The aim of the present invention is to develop a complex *in vitro* model of human skin using 3D bioprinting, where the skin model will comprise all skin layers with cells and will enable *in vitro* testing equivalent to *in vivo* measurements, while addressing most of the drawbacks of existing solutions. It is the first *in vitro* model that additionally comprises a layer of bone *in vitro* tissue that enables measurements and validations of non-invasive sensors. The technical problem is solved as defined in the independent claims, while the preferred embodiments are defined in the dependent claims.

The complex *in vitro* model of human skin according to the invention comprises from the top layer to the bottom layer:
- epidermis with keratinocytes and at least one growth factor enabling proliferation of cells and/or epidermis preferably selected from the group consisting of EGF, FGF, PDGF, wherein EGF is the optimal choice and wherein the preferred concentration of at least one growth factor is comparable to the physiological concentration of the selected growth factor in the skin, preferably 250 pg/ml,
- dermis comprising fibroblasts and at least one growth factor allowing the proliferation of cells, preferably selected from the group consisting of FGF, VEGF, TGF-alfa, TGF-beta, IGF, PLGF, wherein FGF is the optimal choice and wherein the preferred concentration of at least one growth factor is comparable to the physiological concentration of the selected growth factor in the skin, preferably 1000 pg/ml, and preferably further comprising nanofibers of at least one suitable polysaccharide and/or combinations of polysaccharides that simulate the extracellular matrix and thus contribute to improved adhesion to the material and consequently to improved proliferation of cells,
- a subdermal layer comprising:
   ∘ subcutaneous tissue comprising any suitable adipocytes, preferably somatic adipocytes, preferably isolated from human adipose tissue, which influences the mechanical properties and behaviour of active agents during translocation from hollow structures into the surrounding tissue; and
   ∘ hollow structures with endothelial cells arranged to simulate flowable vessels through which flow is enabled by at least one micropump, thereby enabling simulation of blood flow and pressure in vessels, wherein the interior of the hollow structure preferably being provided with at least one pressure sensor, temperature sensor, flow sensor, and/or saturation sensor, enabling measurement of various parameters in blood via the skin and testing of transdermal or dermal preparations (e.g., measurements of the blood concentration of a substance applied to the skin surface), wherein the hollow structures are prepared with any suitable 3D printing technique, preferably core/shell 3D printing and the endothelial cells are incorporated *in situ,* wherein instead of endothelial cells any other suitable cells may be used, for example non-differentiated cells which can later on differentiate into endothelium;
- a bone simulation to allow the use of the skin model for test and measurements, such as the non-invasive measurement of glucose and other human markers that require a solid surface that allows reflection, wherein the bone simulation is achieved with a metal substrate to which a polysaccharide, comprising a combination of alginate, methylcellulose and nanofibrillar cellulose comprising hydroxyapatite, is applied, preferably with 3D printing, wherein human osteoblasts are grown on the polysaccharide under known growth conditions for culturing human osteoblasts.

To ensure prolonged stability, the polymers used in the above-described model are dissolved in cellular media suitable for culturing said cells and provided with antibiotics used according to known protocols for handling *in vitro* cells as known to a person skilled in the art.

The sensors in the interior of the hollow structure may be a part of the in vitro model or a part of the circulation ensured by the pump, wherein connections for connecting the hollow structures to the pump are provided with micro sensors for measurement of various parameters in blood in known manners.

The skin model according to the invention is produced by any suitable method of 3D printing, i.e. extrusion, laser, photolithographic or droplet methods and using a suitable device, preferably an extrusion 3D printer, in combination with electrospinning based on biocompatible polymeric base materials with integrated cells characteristic of selected skin layers. Instead of electrospinning, the simulation of the extracellular matrix (ECM) can be performed by adding nanofibrillar cellulose (NFC).

The method for producing the complex *in vitro* skin model according to the invention comprises the following steps:
a) Preparation of a 3D printed scaffold (framework, skeleton, carrier) with nanofibers to simulate epidermis and dermis, wherein:
   i. the scaffold is 3D printed using a suitable material,
   ii. the simulation of the ECM is performed either by:
      electrospinning nanofibers of a material selected from the group consisting of CMC, alginate, polyethylene oxide, PCL, PVA and combinations thereof, preferably CMC and polyethylene oxide, to form a simulated ECM, wherein suitable conditions for cellular growth are ensured, wherein the polymeric base material can be complemented by an additional material selected from the group consisting of materials that form ECM in the skin, e.g., proteoglycans, hyaluronic acid, collagen, fibronectin), or by adding nanofibrillar cellulose (NFC), which also ensures ECM morphology and a suitable environment to promote proliferation of skin cells,
   iii. addition of fibroblasts and the preferred growth factor, optimally FGF, to form the dermis, the fibroblasts and optional growth factors being added simultaneously with the materials simulating ECM or separately,
   iv. addition of keratinocytes and the preferred growth factor, optimally EGF, to the formed dermis to form a layer of epidermis on top of the already formed dermis,
   v. Preferably repeating steps i to iii, to form a separate second layer of dermis arranged to allow printing of hollow channels,
b) Preparation of a 3D printed flow system to simulate blood flow, where:
   i. Printing hollow structures with a core-shell 3D printer based on a prepared suitable printing file defining the geometry and distribution of the hollow structures,
   ii. adding endothelial cells either in the medium for printing hollow structures or separately so that they can adhere to the already prepared formation of hollow structures,
   iii. incorporating sensors, especially sensors for flow, pressure, temperature, saturation, into the prepared hollow structures,
   iv. addition of fibroblasts for better connection between the layers prepared in steps a) and b) and/or performing said step v) to form a suitably prepared dermis with hollow structures simulating the vascular system.
c) Preparation of subcutaneous tissue, wherein a combination of suitable materials is used for 3D printing, preferably ALG, MC in NFC, with adipocytes,
d) Optional connection of a suitable pump to ensure flow through the simulated vasculature, ensuring connection with a teflon vascular splint installed on a biocompatible silicone tube that allows operation under appropriate pressures and allows circulation of nutrients in the vasculature, and wherein alternative connections using other biocompatible materials are also possible,
e) preparing a metal carrier and 3D printing a suitable polysaccharide or combinations of polysaccharides known to the skilled person, 3D printing a combination of alginate, methylcellulose and nanofibrillar cellulose with hydroxyapatite comprising *in situ* human osteoblasts under suitable conditions and cell media known to the skilled person,
f) Incubating the obtained *in vitro* skin model in a suitable location under suitable conditions and optionally additional proliferation of the prepared tissue.

Alternatively, steps e) to a) are performed in a reverse manner, so that the step e) is the first and the step a) is the last before the incubation step f).

The stability of the *in vitro* model thus produced is at least 30 days, which is suitable for various studies and testing procedures. Due to this determined stability, the in vitro model is considered to be robust and reliable.

Perfusion, which can be altered and therefore used to simulate various (patho)physiological conditions such as increased blood pressure, increased temperature, increased body activity, etc., is performed using peristaltic pumps, micropumps with a maximum flow rate of 100 ml per minute and a size of up to 10 dm³. These pumps are controlled by pulse width modulation. Fine tuning of the pumps is possible via proportional-integral-differential (PID) control systems. For optimal flow control, the PPG signal is used to measure a diastolic and an asystolic peak in the signal that mimics the pulsatile motion of the fluid. To prevent damage to the blood vessels, the flow is limited. In addition, the blood vessel is connected to an outer tube made of suitable material, such as PP, PET and similar, which can be connected to the pump using a vascular splint. This outer tubing does not come into contact with the cells of the *in vitro* model according to the invention, but must still be not-toxic, sterile and suitably prepared, as is known to those skilled in the art. The vascular splint serves as a connection and seal between the natural and the synthetic material, thus preventing possible contamination of the cells by external influences. At the same time, sensors for flow measurement are integrated, said sensors operating as Hall sensors or other non-ultrasonic sensors. In addition, a continuous power supply system is provided, where direct operation from the power grid is possible. This contributes to a more reliable and uninterrupted operation of the pumps.

The 3D structured base material of each layer and the vascular (flow) system is prepared by 3D printing, which may be extrusion, laser, or another process or combination thereof, wherein the pore size and dimensions limited within a certain narrow range, preferably 30 to 1000 µm, which ensures repeatability of the process and simulation of the mechanical properties of human skin. The pore size is defined by the distances between the individual lines and by the angle of rotation for the next layer, which also ensures the repeatability of the preparation process and thus the appearance of the *in vitro* skin model thus prepared according to the invention.

The starting material for the preparation of the 3D base scaffold can be any suitable material known to the person skilled in the art, in particular:
- Natural materials such as:
   ∘ derivatives of polysaccharides - alginate (ALG), carboxymethylcellulose (CMC), nanofibrillar cellulose (NFC), methylcellulose (MC),
   ∘ viscose,
   ∘ silk,
   ∘ collagen, elastin, proteoglycans, aggrecans, glycosaminoglycans (hyaluronic acid), heparin, fibrinogen,
   ∘ o any combination thereof,
- semi-synthetic materials such as
   ∘ chitosan with derivatives,
   ∘ polycaprolactone,
   ∘ any combination thereof,
   - synthetic materials such as:
      ∘ polyethylene terephthalate (PET),
      ∘ polybutylene terephthalate (PBT),
      ∘polypropylene (PP),
      ∘ polyhydroxyethyl methacrylate,
      ∘ polyhydroxybutyl methacrylate,
      ∘ polyvinyl alcohol,
      ∘ polyethylene oxide, and
      ∘ any combination thereof,
- any combination of natural, semi-synthetic and synthetic materials.

All of the above materials are biocompatible, which is known to those skilled in the art.

Preferably, the starting material for the 3D base scaffold is a combination of alginate (ALG), nanofibrillar cellulose (NFC), methyl cellulose (MC) and cellular medium. The preferred ratio between these components is: 10 % MC, 5 % ALG, 1,5 % NFC and crosslinked with 1 to 5 % CaCl₂ or 5 % SrCl₂ or any other suitable cross-linking agent.

The main component of the dermis in physiological environment is the extracellular matrix, which in the present inventions is simulated by nanofibers prepared by electrospinning. In order to achieve high similarity with physiological conditions, the 3D printed scaffold is provided with electrospun nanofibers, wherein the basic polymeric materials for spinning, such as CMC, alginate, polyethylene oxide, PCL, PVA and combinations thereof, are supplemented with materials that form the ECM in the skin, such as proteoglycans, hyaluronic acid, collagen, fibronectin. Preferably, electrospun nanofibers CMC and PEO are used. Alternatively, the ECM structure can be simulated by adding NFC to the hydrogel with cells, ensuring a morphology suitable for promoting skin cell proliferation. The latter method is simpler and the results are comparable to those obtained with electrospinning.

According to the most optimal embodiment the method for preparation of the *in vitro* skin model comprises the following steps:
- the materials ALG, MC, NFC, fibroblasts and FGF are mixed and printed with a 3D printer to obtain a 3D structure of the dermis,
- on the obtained dermis, keratinocytes are added with EGF and incubated for 3 days for proliferation, to allow the formation of epidermis on the dermis layer,
- the materials ALG, MC, NFC and endothelial cells are printed in the form of tubes to simulate the vascular system, around these tubes, a combination of the materials ALG; MC, NFC, fibroblasts and FGF are printed, to obtain simulated blood vessels in the lowermost part of the dermis,
- the materials ALG, MC, NFC and adipocytes are printed under the structure with hollow tubes using a 3D printer to form a layer of subcutaneous/adipose tissue, and
- a combination of ALG, MC, NFC and hydroxyapatite with osteoblasts are printed on the support material, preferably medical grade stainless steel, to simulate bone under the subcutaneous tissue layer.

The thickness of the layers of the *in vitro* skin model can be adjusted, and the preferred layer thicknesses are as follows: epidermis from 80 to 200 µm, dermis from 1500 to 5000 µm, hypodermis (subcutaneous tissue) from 800 to 2500 µm.

The complex *in vitro* skin model according to the invention and the method for preparation thereof enable the production of a structure with similar mechanical properties to human skin, which enables *in vitro* tests that provide similar results to *in vivo* tests. Namely, the skin model according to the invention simulates:
- biochemical properties by using materials that form the ECM in the skin,
- 3D structures with nanofibrillar cellulose simulating the structure of the ECM, and
- physiological properties by simulating blood flow.

The application of the invention is broad, in medicine, pharmaceuticals, biotechnology and other sciences. It has the potential to be used as alternative to *in vivo* studies in testing various materials, chemicals, pharmaceutical preparations, cosmetic preparations, non-invasive sensors and similar products. *In vitro* testing on the complex skin model according to the invention has an obvious advantage over animal and human testing because the results are repeatable and the testing is more efficient, less expensive and also more ethical.

Some of the possible applications are:
- development and testing of novel dermal drugs, e.g. anti-inflammatory dermal drugs, local antibiotics, disinfectants,
- development and testing of transdermal products, such as transdermal patches for pain relief, hormones, etc,
- *in vitro* testing of transdermal pharmaceutical and cosmetic products, e.g. measurement of drug concentration during translocation through the skin surface into the vascular system,
- investigation of the transfer of active substances from the vascular system into the surrounding tissue,
- possibility of measuring parameters such as glucose, glycated haemoglobin, CRP and cholesterol through the skin surface (testing non-invasive measuring devices or sensors for physiological parameters),
- investigation of the effects of physiological influences such as body temperature, cell activity, etc., on the behaviour of the active components or preparations,
- toxicity tests on skin cells and evaluation of biocompatibility of the tested preparations,
- development and testing of a non-invasive sensor to measure glucose, as this skin model allows reliable *in vitro* testing and validation of the sensor event at very low blood glucose levels. The development of non-invasive sensors is progressing rapidly and numerous researchers are looking for solutions for the non-invasive measurement of many physiological parameters (glycated haemoglobin, cholesterol, CRP, etc.). The model according to the invention allows validation of all newly-developed non-invasive sensors.

In the latter case, the invention is particularly advantageous because it allows *in vitro* testing at low blood glucose levels, since it is ethically unacceptable to cause blood glucose levels that are too low for testing purposes, since such conditions can lead to unconsciousness or coma. The accuracy of such measurements at low blood glucose levels is critical, as they may cause unconsciousness or coma in patients suffering from diabetes. The *in vitro* skin model according to the invention is currently the only way to validate non-invasive sensors in the too low blood glucose range, and thus represents an opportunity to develop new safe non-invasive sensors for blood glucose. Since these sensors use a spectroscopic measurement where reflectance is critical, this reflectance is achieved in the presented model by optionally including the bone layer. It is important to obtain reliable and comparable results. Therefore, the addition of the bone layer into the skin model is an important advance as it enables the validation of non-invasive glucose metres or devices measuring other physiological parameters.

The invention will be described in further detail on the basis of exemplary embodiments, examples and figures, which show:
- Figure 1: Schematic depiction of the *in vitro* skin model according to a possible embodiment
- Figure 2: Schematic depiction of the procedure for the preparation of the *in vitro* skin model according to a possible embodiment
- Figure 3: Microscopy results of the *in vitro* model up to day 29 post layer printing

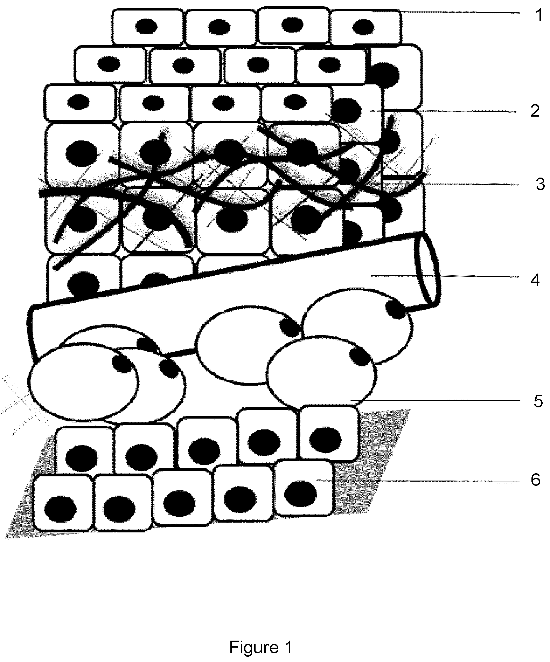

Figure 1 shows a schematic representation of the *in vitro* skin model according to a possible embodiment, comprising the following layers from the uppermost to the lowermost layer:
- epidermis 1 with keratinocytes,
- dermis 2 with fibroblasts,
- extracellular matrix 3,
- flow vessel 4 surrounded with endothelial cells,
- subcutaneous tissue 5 with adipocytes, and
- bone simulation 6 with osteoblasts on a metal substrate.

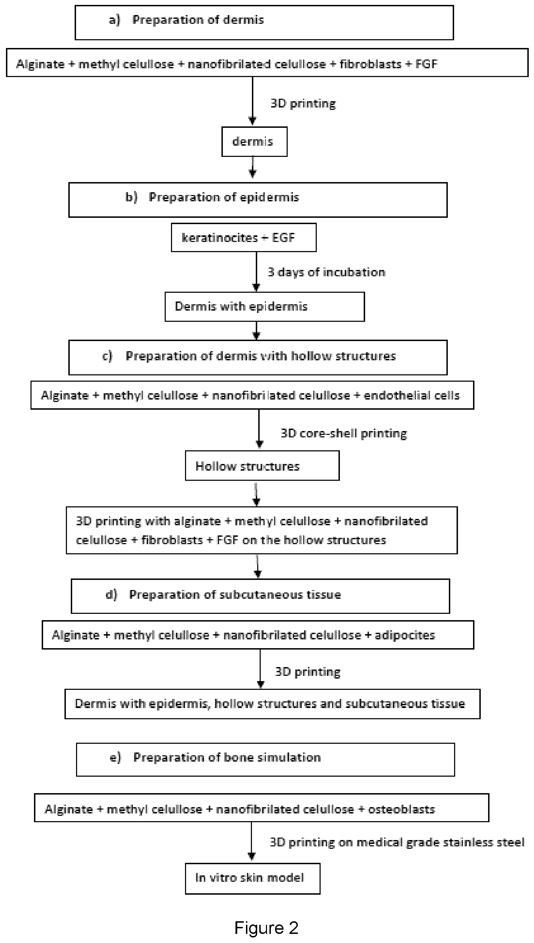

According to the most optimal process shown in figure 2, the embodiment of the *in vitro* skin model shown in figure 1, is performed using the following steps:
a) materials ALG, MC, NFC, fibroblasts and FGF are mixed and printed with a 3D printer to obtain a 3D structure of dermis,
b) onto the obtained dermis, keratinocytes with EGF are provided and incubated for 3 days, to proliferate and allow formation of epidermis on the dermis layer,
c) materials ALG, MC, NFC and endothelial cells are printed in the shape of tubes for simulation of the vascular system, around the said tubes a combination of materials ALG; MC, NFC, fibroblasts and FGF are printed, to obtain simulated blood vessels in the lowermost part of the dermis,
d) materials ALG, MC, NFC and adipocytes are printed with a 3D printer under the structure with hollow tubes, in order to form a layer of subcutaneous tissue, and
e) on the carrier material, preferably medical grade stainless steel, a combination of ALG, MC, NFC and hydroxyapatite with osteoblasts is printed, in order to simulate bone tissue under the layer of subcutaneous tissue.

Using the bellow described examples optimal procedures for the preparation of individual cells and layers forming the *in vitro* model will be discussed. Results of microscopy are shown to prove liveness of cells in the *in vitro* model, which is essential for its operation in studies, analysis and testing.

### Examples

### Example 1: Preparation of dermis with in situ incorporated fibroblasts with epidermis with keratinocytes

### Human skin fibroblast culture for 3D printing

Human skin fibroblasts (hSF) were cultured and maintained in ADMEM supplemented with 5% FBS, 2 mmol/L I-glutamine, 100 U/ml penicillin and 1 mg/ml streptomycin (ADMEM + 5% FBS) at 37°C in a humidified atmosphere with 5% CO₂ in tissue culture plates (NuncTM, Thermo Fisher Scientific, Bremen, Germany) until confluence was reached. The medium was changed every other day. After 3D printing, 3D cell scaffolds with incorporated hSF were cultured in ADMEM + 5% FBS with regular medium changes (every 2-3 days).

Cells were imaged daily with an inverted optical microscope (Axiovert 40, Carl Zeiss Microscopy, Munich, Germany). To assess the *in situ* viability of the incorporated hSFs, Live/Dead staining was performed: 3D cell scaffolds were removed from the medium, washed with PBS (pH 7.4) and treated with Live/Dead solution (according to the manufacturer's protocol). Finally, each stained 3D cell scaffold was observed and imaged using a fluorescence microscope (EVOS FL Cell Imaging System Thermo Fisher Scientific Inc., Germany).

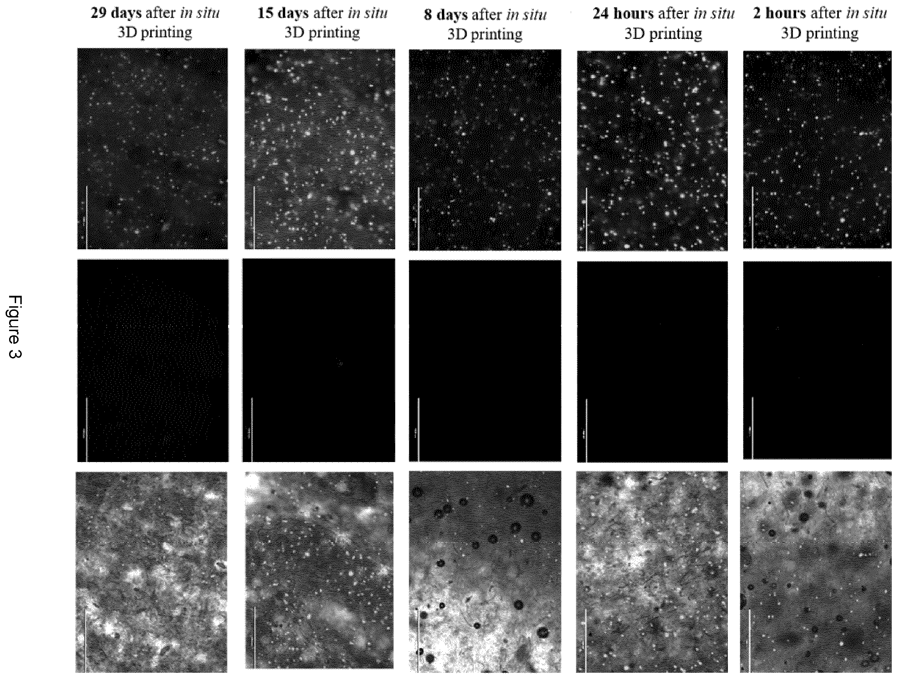

An example of the results is shown in Figure 3 and is further commented below.

### Preparation of bioink (formulation and cells) for 3D printing

gr We fabricated with a layer-by-layer bioprinting process 4-layer 3D printed scaffolds incorporating hSF cells. For this purpose, we used a VitaPrint extrusion 3D bioprinter (IRNAS Institute, Maribor, Slovenia) equipped with nozzles with a diameter of 0.25 mm (Nordson EFD, East Providence, RI, USA), which allows the formation of pores of a selected size and shape. Using a layer-by-layer deposition process on a glass surface,

the scaffold filaments were deposited in a 0/90 rotation for each consecutive layer. Prior to the start of the bioprinting process, a cylinder-shaped template with a diameter of 10 mm and a height of 0,8 mm was modelled using Autodesk software (Autodesk Inc., San Rafael, CA, USA). The pore size was predefined in the g-code generator (Slic3r 1.2.9, open source software released under the GNU Aero General Public License) and was set to a constant 30% infill over the entire surface area of the 3D scaffold.

The formulation used in this study was partially optimised in previous studies [24,36,37]. It was herein further adapted to the specific needs of bioink preparation (the proportion of the aqueous component was replaced by a cell culture medium, a lower concentration of calcium chloride was used for crosslinking, etc.). The bioink preparation procedure was as follows: first, ALG and CMC were mixed in ADMEM + 5% FBS and 3% NFC to give a final formulation of 3% ALG + 3% CMC + 1.5% NFC. The resulting formulation was sterilised under UV light for 30 min. Optimisation of the formulation was necessary to achieve adequate printability and consequently structurally stable scaffolds for the defined geometry. Prior to *in situ* incorporation of the cells into the bioink, hSF cells were resuspended in ADMEM + 5% FBS. Afterwards, 1 mL of hSF suspension (10⁶ cells/mL) was manually mixed into the prepared bioink formulation (ALG/CMC/NFC). After 3D printing, which was performed under sterile conditions in a laminar, all 3D scaffolds with incorporated hSF were subsequently cross-linked with 2% CaCl₂ solution. For cross-linking, the scaffold with cells was soaked in a 2% CaCl₂ solution for 1 min, during which time ionic gelation occurred. The scaffolds were then carefully removed from the solution and transferred to a 12-well plate (P12). Finally, all 3D hSF scaffolds arranged in individual wells of the P12 plate were poured with ADMEM + 5% FBS. The complete P12 plates with 3D printed hSF-loaded scaffolds were immediately transferred to an incubator operated at 37 °C and in a controlled atmosphere with 5% CO₂.

### Example 2: Preparation of vascular tissue with endothelial cells

### Preparation of hydrogels and crosslinking solutions:

- Briefly, ALG and CMC powders are dissolved in ultrapure water with vigorous stirring to give a total of 3% ALG and 3% CMC, the total volume being adjusted accordingly to the sample size and the volume to be printed;
- 1,5% of cellulose nanofibrils (NFC) are added to the ALG-CMC mixture for additional mechanical reinforcement to achieve the desired viscosity suitable for printing;
- The hydrogel suspension shall be mixed at high speed, preferably 6000 rpm, with a mixer until homogeneous so that there are no visible fibres or bubbles in the hydrogel;
- Preparation of 10 ml of a 100 mM solution of calcium chloride (CaCl₂) in ultrapure water to be used as a stock solution for crosslinking during the printing process;
- Preparation of 10 ml of 5% CaCl₂ in ultrapure water to be used as a secondary crosslinking solution for 'post-processing' of the 3D printed scaffolds;

In general, all hydrogel formulations suitable for immediate chemical crosslinking allow one-step hollow tube fabrication and can be used with this type of "core/shell" set-up. The printing and crosslinking mechanisms should be optimised accordingly. The viscosity of the hydrogels will vary according to the desired composition, which can be adjusted by varying polymer concentrations and adding thickening agents (e.g. nanofibres). An ideal viscosity for 3D printing of stable structures means that the extruded fibre is able to retain its shape and hence the scaffold can hold its own weight prior to crosslinking.

### "Core/shell" printing of flow media:

- Before printing, sterilise the bioprinter with a thorough spray of 70% ethanol and expose it to UV-C light for 1 hour.
- Switch on the bioprinter and run the control software included in the 3D printer package.
- Press the **Home** icon to perform the routing procedure.
- Use the **File** | **Import G-Code** toolbar command to import the generated framework g-code.
- Transfer the hydrogel into a sterile syringe (with luer lock attachment) of 5 ml capacity and insert it into one of the extrusion/extrusion ports. Connect it via the luer lock connector and the short tubing to the side port of the luer lock connector of the 'core/shell' nozzle.
- Transfer the cross-linking solution (100 mM CaCl₂) into a second sterile 5 ml syringe with the supplied G27 needle with blunt end and insert it into the upper needle holder of the core/shell nozzle. The inner needle should be slightly protruding (~1 mm) from the outer core/shell nozzle. Adjust the alignment manually. Insert the second syringe into the extrusion/extrusion attachment.
- Manually operate both extrusion/extrusion nozzles by clicking the arrows **A** and **B** and the **Up** and **Down** arrows to insert the syringes correctly into the dedicated nozzles.
- Before starting to print, extrude the hydrogel and the crosslinking solution separately to remove any excess air bubbles in the core/shell nozzle and to ensure a continuous flow of hydrogel.
- Use the **Z** and **Up** and **Down** arrows to manually adjust the distance between the nozzle and the print substrate. It is recommended to use a flat glass substrate that has a good grip. The extrusion nozzle must not be in contact with the substrate to allow a continuous flow of hydrogel. The optimum distance between the nozzle and the substrate (layer height) is normally equal to the width of the outer diameter of the nozzle, but shall be adjusted to the material used and the individual printing parameters. Adjust the initial printing height according to individual needs.
- To start printing, click the **Play** button.
- It is recommended to switch on the printing of the outer skirt around the frame to ensure the laying of a homogeneous hollow filament before the printing of the actual frame starts. Optimum hydrogel flow through the nozzle resulting in printing of optimal scaffolds is achieved by varying the formulation, crosslinking solution composition and adjusting the operational parameters (i.e. printing speed, extrusion/extrusion pressure, printing temperature, substrate-to-nozzle distance, etc.).
- After printing, carefully remove the substrate from the 3D printed scaffold and completely cover it with the secondary crosslinking solution (5 % CaCl₂) to ensure crosslinking over the entire scaffold. Incubate it at room temperature (RT) for 1 minute.
- **NOTE:** Make sure that the printed scaffold is completely covered/immersed in the crosslinking solution. This step is crucial to achieve the desired strength properties of the scaffold, but depends on the material used and the crosslinking method used.
- Using a scalpel, manually cut off the excess outer wing material.
- Sterilise the substrates under UV-C light for 30 min. Carefully invert the frame and repeat the sterilisation process.
- Carefully separate the scaffold from the base by gently pulling it sideways. In case the scaffold is strongly adhering to the substrate, separate them by inserting a thin object with a sharp edge between them.
- Transfer the 3D scaffolds into colourless cell culture medium (DMEM supplemented with 5% FBS, 100 U/mL penicillin and 1 mg/mL streptomycin) and incubate at 37°C in an atmosphere of 5% CO₂ for at least 24 h.

### Preparation of endothelial cell culture and Live/Dead staining solution:

- For cell culture, prepare enriched DMEM (ADMEM) cell culture medium with added phenyl red supplemented with 5% FBS and 2 mM L-glutamine. Add 100 U/mL penicillin and 1 mg/mL streptomycin.
- Establish a human umbilical vein endothelial cell (HUVEC) cell line and perform cell passaging according to the protocol for HUVEC cell line culture as described in HUV-EC-C [HUVEC] (ATCC^{®} CRL-1730^{™}) Homo sapiens umbilical vein. Available at: http://www.lgcstandards-atcc.org/products/all/CRL-1730.aspx?geo_country=si#documentation (Accessed 2019).
- Culturing the cells in phenol red supplemented cell culture medium is recommended to facilitate visualisation of the cell injection into the flow channels within the white translucent scaffolds as described below.
- For cell counting, pipette 100 µl of cells suspended in cell culture medium and stain with 900µl of 0,1% trypan blue solution.

Use an automated cell counter or a manual haemocytometer to count the cells and obtain the estimated number of cells in suspension.

### Transfer the endothelial cells to the 3D scaffold:

- Remove the scaffold from the (colourless) cell culture medium and transfer to a sufficiently large glass petri dish,
- Immediately before starting the injection of cells into the scaffold, detach the HUVEC cells from the walls of the cell culture bottle by using trypsin (0.25%),
- The cell culture medium is removed and incubation of the cells with 0.25 % trypsin (~2 ml) is continued for 5 minutes at 37 °C,
- After incubation, ~3 mL of cell culture medium is added to the trypsinised cells and all detached cells are transferred to a centrifuge,
- Centrifuge the cells at 200 x g for 5 min and remove the supernatant,
- Resuspend the cells in fresh cell culture medium,
- Cell counting and adjustment of the final cell concentration according to individual needs, preferably using an initial concentration of 340,000 cells/mL,
- Resuspension of cells in a sterile syringe with attached G27 needle with blunt end,
- Defining the entry point and injecting the cell suspension into the scaffold to ensure that the entire scaffold is filled with the cell suspension,
- Immersion of the scaffold in cell culture medium and incubation at 37 °C in an atmosphere of 5% CO₂ for up to 10 days,
- Supplementation of the cell culture medium according to the needs of the assay.

### Example 3: Preparation of subcutaneous tissue and adipocytes

### PV-ADMSC

A paravertebral adipose tissue sample was obtained during elective spinal surgery in a 3-month-old child with tethered spinal cord syndrome. Permission for the use of human tissue was obtained from the Medical Ethics Committee of the University Medical Centre Maribor, as well as written informed consent from the patient (UKC-MB-KME-120/13).

Under sterile conditions, adipose tissue fragments of approximately 1 cm² were transferred into phosphate buffered saline (PBS) and transferred to the laboratory. The tissue fragments were transferred from the transport centrifuge tubes into 3,5 cm diameter petri dishes and washed with sterile PBS. The tissue was cut into smaller fragments and incubated with 0,25% trypsin/EDTA mixture (Merck KGaA, Darmstadt, Germany) for 30 min at 37 °C in an atmosphere of 5% CO₂. Enriched DMEM (ADMEM, Thermo Fisher Scientific, MA, USA) comprising 100 IU/ml penicillin, 0,1 mg/ml streptomycin, 2 mM L-glutamine (all Merck KGaA, Darmstadt, Germany) and 5% heat-activated FBS (Thermo Fisher Scientific, MA, USA) was added. The suspension was centrifuged at 200 × g for 5 min. The sediment was resuspended and transferred into T25 cell culture bottles. The growth medium was changed every 3 days. After 14 days of incubation, a monolayer of cells was formed.

### LA-ADMSC

Samples of 50-100 ml of tumescent lipoaspirate were obtained during breast reconstruction procedures in adult women. Written informed consent was obtained from the donors and the study was approved by the National Medical Ethics Committee (approval 0120-349/2018/5).

Within 30 min, lipoaspirate samples were transferred to the cell culture laboratory and processed as described before [Rozanc et al. (Biomedicines 2022, 10(2), 378; https://doi.org/10.3390/biomedicines10020378]. Briefly, samples were washed in Dulbecco's PBS solution to remove blood. This was followed by digestion with collagenase la (Sigma-Aldrich, MI, USA). The samples were then centrifuged and the upper fat layer and supernatant were discarded. The remaining pelleted stromal vascular fraction (SVF) comprising MSCs was resuspended in DMEM/F12 GlutaMAX, supplemented with 10% FBS and 1% Pen/Strep (all Gibco, MA, USA), and filtered through a 70 µm cell strainer to remove undigested tissue debris. The resulting cell suspension was transferred to a cell culture bottle and then cultured in DMEM/F12 GlutaMAX at 37 °C, 5% CO₂ and 90% relative humidity. After one day, the medium was changed to remove unattached cells.

The obtained LA-ADMSC primary cultures were subcultured until passage 4, when they reached ~80% confluency. Cryopreservation at passage 1 and subsequent thawing of the cells were performed following the procedure described before [Rozanc et al. (Biomedicines 2022, 10(2), 378; https://doi.org/10.3390/biomedicines10020378].

### PCS-500-011

The normal human (human) adipose tissue-derived mesenchymal stem cell line PCS-500-011 was purchased from ATTC (ATTC, MA, USA). The cells were grown in mesenchymal stem cell basic medium (MSCBM) with the added components provided in the low serum mesenchymal stem cell growth kit (2% FBS, 5 ng/ml rh FGF basic, 5 ng/ml rh FGF acidic, 5 ng/ml rh EGF, 2.4 mM L-alanyl-L-glutamine). (Cells were grown in a humidified incubator at 37 °C and 5% CO₂. Handling, sub-culturing and maintenance of the cultures were carried out according to the manufacturer's instructions.

### Example 4: Preparation of osteoblasts and bone simulation

Osteoblasts for the preparation of the in vitro model can be obtained in many ways, but the following steps are preferred:
a) In the operating room, the surgeon breaks the bone into the smallest possible pieces,
b) The bone pieces obtained in step a) shall be placed in Advanced DMEM or saline or PBS medium, with a short incubation, if necessary, until isolation in the next step begins,
c) washing the bone pieces from step b) with PBS to obtain a clear medium free of blood admixture by vortexing the pieces in PBS, allowing them to sediment, then discarding the PBS, pouring fresh PBS, vortexing, and repeating the above steps until the medium is clear,
d) 15 ml of Advanced DMEM supplemented with 5% FBS is poured into the cell culture petri dish and the bone pieces washed in the previous step are placed in the petri dish using tweezers,
e) Incubate the bone pieces in the petri dish from step d) at 37°C for approximately one week to allow osteoblasts to grow out of the bone pieces,
f) trypsinisation of the cells generated in step e) and transfer of the bone pieces into a new petri dish with fresh Advanced DMEM with 5% FBS (i.e. replating).

The cells thus prepared are then embedded in a hydrogel of methyl cellulose (MC), alginate (ALG), hydroxyapatite and nanofibrillated cellulose (NFC) and then printed in *situ* on a medical grade stainless steel or titan support using a 3D printer to achieve bone simulation.

The exemplary protocol for preparation of the in vitro model according to example 4
- preparing a metal carrier, i.e., medical steel or titan, and 3D printing a combination of alginate, methylcellulose and nanofibrillar cellulose with hydroxyapatite comprising *in situ* human osteoblasts under suitable conditions and cell media known to the skilled person,
- Preparation of subcutaneous tissue, wherein a combination of suitable materials is used for 3D printing, preferably ALG, MC in NFC, with adipocytes,
- Preparation of a 3D printed flow system to simulate blood flow, where:
   ∘ Printing hollow structures with a core-shell 3D printer based on a prepared suitable printing file defining the geometry and distribution of the hollow structures,
   ∘ adding endothelial cells either in the medium for printing hollow structures or separately so that they can adhere to the already prepared formation of hollow structures,
   ∘ incorporating sensors, especially sensors for flow, pressure, temperature, saturation, into the prepared hollow structures,
   ∘ addition of fibroblasts form a suitably prepared dermis with hollow structures simulating the vascular system,
- Preparation of a 3D printed scaffold (framework, skeleton, carrier) with nanofibers to simulate epidermis and dermis, wherein:
   ∘ the scaffold is 3D printed using a suitable material,
   ∘ the simulation of the ECM is performed either by:
      ∘ electrospinning nanofibers of a material selected from the group consisting of CMC, alginate, polyethylene oxide, PCL, PVA and combinations thereof, preferably CMC and polyethylene oxide, to form a simulated ECM, wherein suitable conditions for cellular growth are ensured, wherein the polymeric base material can be complemented by an additional material selected from the group consisting of materials that form ECM in the skin, e.g., proteoglycans, hyaluronic acid, collagen, fibronectin), or by adding nanofibrillar cellulose (NFC), which also ensures ECM morphology and a suitable environment to promote proliferation of skin cells,
      ∘ addition of fibroblasts and the preferred growth factor, optimally FGF, to form the dermis, the fibroblasts and optional growth factors being added simultaneously with the materials simulating ECM or separately,
      ∘ addition of keratinocytes and the preferred growth factor, optimally EGF, to the formed dermis to form a layer of epidermis on top of the already formed dermis,
      ∘ Preferably repeating previous steps,
- Incubating the obtained *in vitro* skin model in a suitable location under suitable conditions and optionally additional proliferation of the prepared tissue.

### Example 5: Analysis of liveness of cells in the in vitro skin model prepared according to the procedures presented in the above examples

Figure 3 shows the microscopy results of the prepared *in vitro* model, namely the dermis layer with fibroblasts up to day 29 after 3D printing of the layer. On the left is a green fluorescence view showing live cells (green), in the middle is red fluorescence where red dots can be seen which are dead cells, while the third column shows the combined fluorescence images with the ones obtained under white light (combination of green and red fluorescence images with brightfield images). It can be seen from the image that two hours after printing, the majority of the cells are alive, and that they continue to multiply over time, and in particular, the vast majority remain alive even 29 days after printing the layer, which means that the prepared material is stable over time and also allows for reliable testing, as there is almost no observed dead cells. This is confirmed by the red fluorescence results, where the number of red-stained, i.e. dead, cells is negligibly small and does not increase with time.

## Claims

1. A complex *in vitro* model of human skin, wherein the model comprises the following layers from the uppermost to the lowest:
- epidermis with keratinocytes provided with at least one growth factor that enables proliferation of cells or epidermis, respectively, said growth factor being selected in the group consisting of EGF, FGF, PDGF,
- dermis with fibroblasts and simulation of extracellular matrix with at least one of suitable polysaccharides and/or combinations thereof, wherein said fibroblasts are provided with at least one growth factor selected in the group consisting of FGF, VEGF, TGF-alfa, TGF-beta, IGF, PLGF,
wherein said epidermis in dermis is prepared in the following manner:
- preparation of a 3D printed scaffold with nanofibers for simulation of epidermis and dermis, wherein:
- the scaffold is 3D printed using a suitable material,
- Simulation of ECM is performed either by:
∘ electrospinning of nanofibers from a material selected in the group consisting of CMC, alginate, polyethylene oxide, PCL, PVA and combinations thereof, preferably CMC and polyethylene oxide, to form a simulated ECM, wherein suitable conditions for cellular growth are ensured, wherein the basic polymeric material may be supplemented with an additional material selected in the group consisting of materials that form ECM in skin, for example proteoglycans, hyaluronic acid, collagen, fibronectin), or
∘ by addition of nanofibrillar cellulose (NFC), which also ensures ECM morphology and a suitable environment for enhancing proliferation of skin cells,
- addition of fibroblasts and the growth factor selected in the group consisting of FGF, VEGF, TGF-alfa, TGF-beta, IGF, PLGF, to form dermis, wherein the fibroblasts and growth factors are added simultaneously with the materials for simulation of ECM or separately,
- Addition of keratinocytes and the growth factor selected in the group consisting of EGF, FGF, PDGF, optimally EGF, to the formed dermis, in order to form a layer of epidermis on the already formed dermis,
- Preferably repeating steps i to iii, to form a separate second layer of dermis arranged to allow printing of hollow channels,
- a subdermal layer comprising:
∘ subcutaneous tissue with adipocytes, preferably somatic adipocytes, and
∘ hollow structures with endothelial cells for simulation of blood vessels, wherein flow through said hollow structures is enabled with at least one micropump simulating blood flow and pressure inside the hollow structures, wherein the interior of the hollow structures is provided with at least one sensor arranged to measure flow, pressure, temperature and/or saturation, and wherein said hollow structures are prepared using 3D printing, preferably core/shell 3D printing and cells,
wherein the subdermal layer is prepared in the following manner:
- Hollow structures printed with core-shell 3D printing based on a pre-prepared suitable printing file, which defines the geometry and distribution of hollow structures,
- Addition of endothelial cells either in the medium for printing hollow structures or separately, so that they can adhere to the already prepared formation of hollow structures,
- Installation of sensors, particularly sensors for flow, pressure, temperature, saturation, into the prepared hollow structures,
- Addition of fibroblasts for improved connection between layers prepared in steps a) and b) and/or performing said step v), to form a suitably prepared dermis with hollow structures simulating the vascular system,
and wherein under subdermal layer a bone simulation layer is provided, said bone simulation layer comprising a metal substrate and a polysaccharide layer comprising a combination of alginate, methylcellulose and nanofibrillar cellulose comprising hydroxyapatite, applied on said metal substrate, wherein human osteoblasts cultivated on the said polysaccharide layer.

2. The complex *in vitro* human skin model according to claim 1, wherein adipocytes are isolated from human fat tissue.

3. The complex *in vitro* human skin model according to any of the preceding claims, wherein the thickness of individual layers of the *in vitro* skin morel are arbitrary, and wherein the preferred thicknesses are: for epidermis from 80 to 200 µm, for dermis from 1500 to 5000 µm, for hypodermis (subcutaneous tissue) from 800 to 2500 µm.

4. The complex *in vitro* human skin model according to any of the preceding claims, wherein the skin model is prepared with any suitable 3D printing process, namely extrusion, laser, photolithographic or droplet process, preferably with extrusion 3D printing.

5. The complex *in vitro* human skin model according to any of the preceding claims, wherein simulation of extracellular matrix is achieved with electrospun biocompatible polymeric carrier materials with integrated cells, or addition of nanofibrillar cellulose (NFC).

6. The complex *in vitro* human skin model according to any of the preceding claims, wherein the said polysaccharide for simulation of extracellular matrix is selected in a group consisting of:
- Natural materials such as:
∘ Derivates of polysaccharides - alginate (ALG), carboxymethyl cellulose (CMC), nanofibrillar cellulose (NFC), methyl cellulose (MC),
∘ viscose,
∘ silk,
∘ collagen, elastin, proteoglycans, aggrecans, glycosaminoglycans (hyaluronic acid), heparin, fibrinogen,
∘ any combination thereof,
- semi-synthetic materials such as
∘ chitosan with derivatives,
∘ polycaprolactone,
∘ any combination thereof,
- synthetic materials such as:
∘ polyethylene terephthalate (PET),
∘ polybutylene terephthalate (PBT),
∘ polypropylene (PP),
∘ polyhydroxyethyl methacrylate,
∘ polyhydroxybutyl methacrylate,
∘ polyvinyl alcohol,
∘ polyethyleneoxyde, and
∘ any combination thereof,
- any combination of natural, semi-synthetic and synthetic materials.

7. The complex *in vitro* human skin model according to any of the preceding claims, wherein for simulation of extracellular matrix and bone simulation a polysaccharide combination comprising alginate, methylcellulose, nanofibrillar cellulose (NFC) with hydroxyapatite is used, wherein the preferred amounts are:
- 10 % methylcellulose,
- 5 % alginate,
- 1,5 % NFC and
- 1 to 5% CaCl₂ or 5 % SrCl₂.

8. The complex *in vitro* human skin model according to any of the preceding claims, wherein perfusion is achieved with peristaltic pumps, micropumps with maximal flow of 100 ml per minute and size up to 10 dm³, wherein said pumps are controlled using pulse width modulation.

9. The complex *in vitro* human skin model according to any of the preceding claims, wherein the hollow structure is connected to an external tube made form a suitable material, such as PP, PET, which may be connected to the pump using a vascular splint, wherein said tube is not in contact with cells of the *in vitro* model, but have to be non-toxic and sterile, wherein said vascular splint serves as a connection and a seal between the hollow structure and the tube and prevents contamination of cells due to external conditions.

10. A process for preparation of the complex *in vitro* human skin model according to any of the preceding claims, **characterized in that** the process comprises the following steps:
a) preparation of 3D printed scaffold with nanofibers for simulation of epidermis and dermis, wherein:
i. the framework is 3D printed using a suitable material,
ii. Simulation of ECM is performed either by:
electrospinning of nanofibers from a material selected in the group consisting of CMC, alginate, polyethylene oxide, PCL, PVA and combinations thereof, preferably CMC and polyethylene oxide, to form a simulated ECM, wherein suitable conditions for cellular growth are ensured, wherein the basic polymeric material may be supplemented with an additional material selected in the group consisting of materials that form ECM in skin, for example proteoglycans, hyaluronic acid, collagen, fibronectin), or
by addition of nanofibrillar cellulose (NFC), which also ensures ECM morphology and a suitable environment for enhancing proliferation of skin cells,
iii. Addition of fibroblasts and a growth factor selected in the group consisting of FGF, VEGF, TGF-alfa, TGF-beta, IGF, PLGF, to form dermis, wherein the fibroblasts and growth factors are added simultaneously with the materials for simulation of ECM or separately,
iv. Addition of keratinocytes and a growth factor selected in the group consisting of EGF, FGF, PDGF, to the formed dermis, in order to form a layer of epidermis on the already formed dermis,
v. Preferably repeating steps i to iii, to form a separate second layer of dermis arranged to allow printing of hollow channels,
b) Preparation of 3D printed flow system for simulation of blood flow, wherein:
i. Hollow structures are printed with core-shell 3D printing based on a pre-prepared suitable printing file, which defines the geometry and distribution of hollow structures,
ii. Addition of endothelial cells either in the medium for printing hollow structures or separately, so that they can adhere to the already prepared formation of hollow structures,
iii. Installation of sensors, particularly sensors for flow, pressure, temperature, saturation, into the prepared hollow structures,
iv. Addition of fibroblasts for improved connection between layers prepared in steps a) and b) and/or performing said step v), to form a suitably prepared dermis with hollow structures simulating the vascular system,
c) Preparation of subcutaneous tissue, wherein a combination of suitable materials is used for 3D printing, preferably ALG, MC in NFC, with adipocytes,
d) Optionally, connecting at least one suitable pump for ensuring flow through the simulated vascular system, wherein the connection is ensured with a Teflon vascular splint installed on a silicone biocompatible tube that enables operation under suitable pressures and enables circulation of nutrients in the vascular system, and wherein alternative connections using other biocompatible materials are also possible,
e) preparing a metal substrate and 3D printing of a polysaccharide layer comprising a combination of alginate, methylcellulose and nanofibrillar cellulose with hydroxyapatite, comprising in situ human osteoblasts,
f) Incubation of the obtained *in vitro* skin model in a suitable place at suitable conditions and optionally additional proliferation of the prepared tissue.

11. The process according to claim 14 or 15, **characterized in that** it comprises the following steps:
- Materials ALG, MC, NFC, fibroblasts and the growth factor FGF are mixed and printed with a 3D printer to obtain a 3D structure of dermis,
- Onto the obtained dermis keratinocytes with the growth factor EGF are provided and incubated for proliferation for 3 days, to allow formation of epidermis on the dermis layer,
- Materials ALG, MC, NFC and endothelial cells are printed in the shape of tubes for simulation of the vascular system, around the said tubes a combination of materials ALG; MC, NFC, fibroblasts and the growth factor FGF are printed, to obtain simulated blood vessels in the lowermost part of the dermis,
- Materials ALG, MC, NFC and adipocytes are printed with a 3D printer under the structure with hollow tubes, in order to form a layer of subcutaneous tissue, and
- the carrier material, is medical grade stainless steel,.

12. In vitro use of the complex *in vitro* human skin model according to any claim from 1 to 9 in medicine, pharmacy, biotechnology and in testing of materials, chemicals, pharmaceutical agents, cosmetic products and similar compounds, preferably in:
- Development and testing of novel dermal drugs, for example anti-inflammatory dermal drugs, local antibiotics, disinfection agents, and/or
- Development and testing of transdermal products, such as transdermal patches for pain relief, hormones, etc., and/or
- *in vitro* testing of transdermal pharmaceutical and cosmetic preparations, for example measurement of active agent concentration upon translocation through skin surface to the vascular system, and/or
- studying transfer of active substances from the vascular system into the surrounding tissue, and/or
- possibility of measuring parameters such as glucose, glycated haemoglobin, CRP and cholesterol via skin surface, and/or
- studying the effect of physiologic influences, such as body temperature, cell activity, etc., on the behaviour of tested active components or preparations, and/or
- toxicity testing on the skin cells and evaluation of biocompatibility of tested preparations, and/or
- Development and testing of non-invasive sensor for measuring glucose or other non-invasive sensors, measuring properties through or on the skin.

## Patentansprüche

1. Komplexes *In-vitro-Modell* der menschlichen Haut, wobei das Modell die folgenden Schichten von der obersten zur untersten umfasst:
- Epidermis mit Keratinozyten, die mit mindestens einem Wachstumsfaktor versehen sind, der die Proliferation von Zellen bzw. der Epidermis ermöglicht, wobei dieser Wachstumsfaktor aus der Gruppe bestehend aus EGF, FGF, PDGF ausgewählt ist,
- Dermis mit Fibroblasten und Simulation der extrazellulären Matrix mit mindestens einem geeigneten Polysaccharid und/oder Kombinationen davon, wobei die Fibroblasten mit mindestens einem Wachstumsfaktor aus der Gruppe bestehend aus FGF, VEGF, TGF-α, TGF-β, IGF, PLGF versehen sind,
wobei die Epidermis in der Dermis auf folgende Weise präpariert wird:
- Präparation eines 3D-gedruckten Zellträgers mit Nanofasern zur Simulation von Epidermis und Dermis, wobei:
- der Zellträger unter Verwendung eines geeigneten Materials 3D-gedruckt ist,
- die ECM-Simulation ausgeführt wird entweder durch:
∘ Elektrospinnen von Nanofasern aus einem Material, ausgewählt aus der Gruppe bestehend aus CMC, Alginat, Polyethylenoxid, PCL, PVA und Kombinationen davon, vorzugsweise CMC und Polyethylenoxid, um eine simulierte ECM zu bilden, wobei geeignete Bedingungen für Zellwachstum gewährleistet sind, wobei das basische Polymermaterial mit einem zusätzlichen Material ergänzt werden kann, das aus der Gruppe ausgewählt ist, bestehend aus Materialien, die ECM in der Haut bilden, zum Beispiel Proteoglykane, Hyaluronsäure, Kollagen, Fibronektin), oder
∘ durch Zugabe von nanofibrillärer Cellulose (NFC), die auch die ECM-Morphologie und ein geeignetes Umfeld zum Fördern der Proliferation von Hautzellen gewährleistet,
- Zugabe von Fibroblasten und dem ausgewählten Wachstumsfaktor aus der Gruppe bestehend aus FGF, VEGF, TGF-α, TGF-β, IGF, PLGF zur Bildung der Dermis, wobei die Fibroblasten und Wachstumsfaktoren gleichzeitig mit den Materialien zur Simulation der extrazellulären Matrix oder separat hinzugefügt werden,
- Zugabe von Keratinozyten und dem ausgewählten Wachstumsfaktor in der Gruppe bestehend aus EGF, EFG, PDGF, optimalerweise EGF, zu der gebildeten Dermis, um eine Epidermisschicht auf der bereits gebildeten Dermis zu bilden,
- vorzugsweise Wiederholen der Schritte i bis iii, um eine separate zweite Dermisschicht zu bilden, die angeordnet ist, um das Drucken von Hohlkanälen zu ermöglichen,
- wobei eine subdermale Schicht Folgendes umfasst:
∘ subkutanes Gewebe mit Adipozyten, vorzugsweise somatischen Adipozyten, und
∘ Hohlstrukturen mit Endothelzellen zur Simulation von Blutgefäßen, wobei der Durchfluss durch die Hohlstrukturen mit mindestens einer Mikropumpe ermöglicht wird, die den Blutfluss und Druck innerhalb der Hohlstrukturen simuliert, wobei das Innere der Hohlstrukturen mit mindestens einem Sensor versehen ist, der angeordnet ist, um Durchfluss, Druck, Temperatur und/oder Sättigung zu messen, und wobei die Hohlstrukturen unter Verwendung von 3D-Druck, vorzugsweise Kern/Hülle-3D-Druck, präpariert werden und Zellen,
wobei die subdermale Schicht auf folgende Weise präpariert wird:
- Hohlstrukturen gedruckt mit Core-Shell-3D-Druck, basierend auf einer vorgefertigten geeigneten Druckdatei, welche die Geometrie und Verteilung von Hohlstrukturen definiert,
- Zugabe von Endothelzellen entweder in das Medium zum Drucken von Hohlstrukturen oder separat, sodass sie an der bereits präparierten Formation von Hohlstrukturen haften können,
- Einbau von Sensoren, insbesondere Sensoren für Durchfluss, Druck, Temperatur, Sättigung, in die präparierten Hohlstrukturen,
- Zugabe von Fibroblasten zur verbesserten Verbindung zwischen den in den Schritten a) und b) präparierten Schichten und/oder Durchführung des Schritts v), um eine geeignet präparierte Dermis mit Hohlstrukturen zu bilden, die das Gefäßsystem simulieren,
und wobei unter der subkutanen Schicht eine Knochensimulationsschicht vorgesehen ist, wobei die Knochensimulationsschicht ein Metallsubstrat und eine Polysaccharidschicht umfasst, die eine Kombination aus Alginat, Methylcellulose und nanofibrillärer Cellulose mit Hydroxylapatit umfasst, die auf dem Metallsubstrat aufgebracht ist, wobei menschliche Osteoblasten auf der Polysaccharidschicht kultiviert werden.

2. Das komplexe *In-vitro-Modell* der menschlichen Haut nach Anspruch 1, wobei Adipozyten aus menschlichem Fettgewebe isoliert werden.

3. Das komplexe *In-vitro-Modell* der menschlichen Haut nach einem der vorstehenden Ansprüche, wobei die Dicke der einzelnen Schichten der *In-vitro-*Hautmorchel willkürlich ist, und wobei die bevorzugten Dicken sind: für Epidermis von 80 bis 200 µm, für Dermis von 1500 bis 5000 µm, für Hypodermis (subkutanes Gewebe) von 800 bis 2500 µm.

4. Das komplexe *In-vitro-Modell* der menschlichen Haut nach einem der vorstehenden Ansprüche, wobei das Hautmodell mit einem geeigneten 3D-Druckverfahren, d. h. Extrusions-, Laser-, Fotolithografie- oder Tröpfchenverfahren, vorzugsweise mit Extrusions-3D-Druck, präpariert wird.

5. Das komplexe *In-vitro-Modell* der menschlichen Haut nach einem der vorstehenden Ansprüche, wobei die Simulation der extrazellulären Matrix durch elektrogesponnene biokompatible polymere Trägermaterialien mit integrierten Zellen oder durch Zugabe von nanofibrillärer Cellulose (NFC) erreicht wird.

6. Das komplexe *In-vitro-Modell* der menschlichen Haut nach einem der vorstehenden Ansprüche, wobei das Polysaccharid zur Simulation der extrazellulären Matrix aus einer Gruppe ausgewählt ist, die aus Folgendem besteht:
- natürlichen Materialien wie:
∘ Derivate von Polysacchariden - Alginat (ALG), Carboxymethylcellulose (CMC), nanofibrilläre Cellulose (NFC), Methylcellulose (MC),
∘ Viskose,
∘ Seide,
∘ Kollagen, Elastin, Proteoglykane, Aggrekane, Glykosaminoglykane (Hyaluronsäure), Heparin, Fibrinogen,
∘ jeder beliebigen Kombination davon,
- halbsynthetischen Materialien wie
∘ Chitosan mit Derivaten,
∘ Polycaprolacton,
∘ jeder beliebigen Kombination davon,
- synthetischen Materialien wie:
∘Polyethylenterephthalat (PET),
∘ Polybutylenterephthalat (PBT),
∘ Polypropylen (PP),
∘ Polyhydroxyethylmethacrylat,
∘ Polyhydroxybutylmethacrylat,
∘ Polyvinylalkohol,
∘ Polyethylenoxid und
∘ jeder beliebigen Kombination davon,
- jeder beliebiger Kombination aus natürlichen, halbsynthetischen und synthetischen Materialien.

7. Das komplexe *In-vitro-Modell* der menschlichen Haut nach einem der vorstehenden Ansprüche, wobei zur Simulation der extrazellulären Matrix und Knochensimulation eine Polysaccharidkombination, die Alginat, Methylcellulose, nanofibrilläre Cellulose (NFC) mit Hydroxylapatit umfasst, verwendet wird, wobei die bevorzugten Mengen sind:
- 10 % Methylcellulose,
- 5 % Alginat,
- 1,5 % NFC und
- 1 bis 5 % CaCl₂ oder 5 % SrCl₂.

8. Das komplexe *In-vitro-Modell* der menschlichen Haut nach einem der vorstehenden Ansprüche, wobei die Perfusion mit peristaltischen Pumpen, Mikropumpen mit einem maximalen Durchfluss von 100 ml pro Minute und einer Größe von bis zu 10 dm³ erreicht wird, wobei die Pumpen unter Verwendung von Pulsweitenmodulation gesteuert werden.

9. Das komplexe *In-vitro-Modell* der menschlichen Haut nach einem der vorstehenden Ansprüche, wobei die Hohlstruktur mit einem externen Schlauch aus einem geeigneten Material, wie PP oder PET, verbunden ist, der unter Verwendung einer Gefäßschiene mit der Pumpe verbunden sein kann, wobei der Schlauch nicht mit den Zellen des In-vitro-Modells in Kontakt kommt und ungiftig und steril sein muss, wobei die Gefäßschiene als Verbindung und Abdichtung zwischen der Hohlstruktur und dem Schlauch dient und eine Kontamination der Zellen durch äußere Bedingungen verhindert.

10. Verfahren der Präparation des komplexen In-vitro-Modells der menschlichen Haut nach einem der vorstehenden Ansprüche, **dadurch gekennzeichnet, dass** das Verfahren die folgenden Schritte umfasst:
a) Präparation eines 3D-gedruckten Zellträgers mit Nanofasern zur Simulation von Epidermis und Dermis, wobei:
i. das Gestell unter Verwendung eines geeigneten Materials 3D-gedruckt ist,
ii. die ECM-Simulation ausgeführt wird entweder durch:
Elektrospinnen von Nanofasern aus einem Material, ausgewählt aus der Gruppe bestehend aus CMC, Alginat, Polyethylenoxid, PCL, PVA und Kombinationen davon, vorzugsweise CMC und Polyethylenoxid, um eine simulierte ECM zu bilden, wobei geeignete Bedingungen für Zellwachstum gewährleistet sind, wobei das basische Polymermaterial mit einem zusätzlichen Material ergänzt werden kann, das aus der Gruppe ausgewählt ist, bestehend aus Materialien, die ECM in der Haut bilden, zum Beispiel Proteoglykane, Hyaluronsäure, Kollagen, Fibronektin), oder
durch Zugabe von nanofibrillärer Cellulose (NFC), die auch die ECM-Morphologie und ein geeignetes Umfeld zum Fördern der Proliferation von Hautzellen gewährleistet,
iii. Zugabe von Fibroblasten und einem Wachstumsfaktor, ausgewählt aus der Gruppe bestehend aus FGF, VEGF, TGF-α, TGF-β, IGF, PLGF zur Bildung der Dermis, wobei die Fibroblasten und Wachstumsfaktoren gleichzeitig mit den Materialien zur Simulation der extrazellulären Matrix oder separat hinzugefügt werden,
iv. Zugabe von Keratinozyten und einem Wachstumsfaktor, ausgewählt aus der Gruppe bestehend aus EGF, EFG, PDGF zu der gebildeten Dermis, um eine Epidermisschicht auf der bereits gebildeten Dermis zu bilden, v. vorzugsweise Wiederholen der Schritte i bis iii, um eine separate zweite Dermisschicht zu bilden, die angeordnet ist, um das Drucken von Hohlkanälen zu ermöglichen,
b) Präparation eines 3D-gedruckten Strömungssystems zur Simulation des Blutflusses, wobei:
i. Hohlstrukturen mit Core-Shell-3D-Druck gedruckt werden, basierend auf einer vorgefertigten geeigneten Druckdatei, welche die Geometrie und Verteilung von Hohlstrukturen definiert,
ii. Zugabe von Endothelzellen entweder in das Medium zum Drucken von Hohlstrukturen oder separat, sodass sie an der bereits präparierten Formation von Hohlstrukturen haften können,
iii. Einbau von Sensoren, insbesondere Sensoren für Durchfluss, Druck, Temperatur, Sättigung, in die präparierten Hohlstrukturen,
iv. Zugabe von Fibroblasten zur verbesserten Verbindung zwischen den in den Schritten a) und b) präparierten Schichten und/oder Durchführung des Schritts v), um eine geeignet präparierte Dermis mit Hohlstrukturen zu bilden, die das Gefäßsystem simulieren,
c) Präparation von subkutanem Gewebe, wobei eine Kombination geeigneter Materialien für den 3D-Druck verwendet wird, vorzugsweise ALG, MC in NFC, mit Adipozyten,
d) optional Anschließen mindestens einer geeigneten Pumpe zum Sicherstellen des Durchflusses durch das simulierte Gefäßsystem, wobei das Anschließen mit einer Teflon-Gefäßschiene sichergestellt wird, die auf einem biokompatiblen Silikonschlauch installiert ist, der einen Betrieb unter geeigneten Drücken und eine Zirkulation von Nährstoffen im Gefäßsystem ermöglicht, und wobei auch alternative Verbindungen mit anderen biokompatiblen Materialien möglich sind,
e) Präparieren eines Metallsubstrats und 3D-Druck einer Polysaccharidschicht, umfassend eine Kombination aus Alginat, Methylcellulose und nanofibrillärer Cellulose mit Hydroxylapatit, umfassend menschliche In-situ-Osteoblasten,
f) Inkubation des erhaltenen In-vitro-Hautmodells an einem geeigneten Ort unter geeigneten Bedingungen und optional zusätzliche Proliferation des präparierten Gewebes.

11. Das Verfahren nach Anspruch 14 oder 15, **dadurch gekennzeichnet, dass** es die folgenden Schritte umfasst:
- Materialien ALG, MC, NFC, Fibroblasten und der Wachstumsfaktor FGF werden gemischt und mit einem 3D-Drucker gedruckt, um eine 3D-Struktur der Dermis zu erhalten.
- Auf die erhaltene Dermis werden Keratinozyten mit dem Wachstumsfaktor EGF bereitgestellt und zur Proliferation 3 Tage lang inkubiert, um die Bildung von Epidermis auf der Dermisschicht zu ermöglichen.
- Materialien ALG, MC, NFC und Endothelzellen werden in Form von Röhren zur Simulation des Gefäßsystems gedruckt, um die Röhren herum wird eine Kombination der Materialien ALG, MC, NFC, Fibroblasten und dem Wachstumsfaktor FGF gedruckt, um simulierte Blutgefäße im untersten Teil der Dermis zu erhalten.
- Materialien ALG, MC, NFC und Adipozyten werden mit einem 3D-Drucker unter die Struktur mit Hohlröhren gedruckt, um eine Schicht von subkutanem Gewebe zu bilden, und
- das Trägermaterial ist medizinischer Edelstahl.

12. *In-vitro*-Verwendung des komplexen In-vitro-Hautmodells der menschlichen Haut nach einem der Ansprüche 1 bis 9 in Medizin, Pharmazie, Biotechnologie und bei der Erprobung von Materialien, Chemikalien, pharmazeutischen Wirkstoffen, kosmetischen Produkten und ähnlichen Verbindungen, vorzugsweise in:
- Entwicklung und Erprobung neuartiger dermaler Arzneimittel, beispielsweise entzündungshemmender dermaler Arzneimittel, lokaler Antibiotika, Desinfektionsmittel, und/oder
- Entwicklung und Erprobung transdermaler Produkte wie transdermaler Pflaster zur Schmerzlinderung, für Hormone usw. und/oder
- *In-vitro*-Erprobung transdermaler pharmazeutischer und kosmetischer Zubereitungen, beispielsweise Messung der Wirkstoffkonzentration bei Translokation durch die Hautoberfläche zum Gefäßsystem, und/oder
- Untersuchung des Transfers von Wirkstoffen aus dem Gefäßsystem in das umliegende Gewebe, und/oder
- Möglichkeit der Messung von Parametern wie Glukose, glykiertem Hämoglobin, CRP und Cholesterin über die Hautoberfläche und/oder
- Untersuchung der Wirkung physiologischer Einflüsse, wie Körpertemperatur, Zellaktivität usw. auf das Verhalten getesteter Wirkstoffe oder Präparate und/oder
- Toxizitätsprüfungen an den Hautzellen und Bewertung der Biokompatibilität der getesteten Präparate und/oder
- Entwicklung und Erprobung nicht-invasiver Sensoren zur Messung von Glukose oder anderer nicht-invasiver Sensoren, die Merkmale durch die oder auf der Haut messen.

## Revendications

1. Modèle complexe *in vitro* de peau humaine, procédé de préparation et utilisation de celui-ci, où le modèle comprend les couches suivantes, de la plus haute à la plus basse :
- épiderme avec kératinocytes pourvus d'au moins un facteur de croissance qui permet la prolifération des cellules ou de l'épiderme, respectivement, ledit facteur de croissance étant sélectionné dans le groupe comprenant l'EGF, le FGF, le PDGF,
- derme avec fibroblastes et simulation de matrice extracellulaire (ECM) avec au moins un type de polysaccharides et/ou des combinaisons de polysaccharides appropriés, où lesdits fibroblastes sont pourvus d'au moins un facteur de croissance sélectionné dans le groupe comprenant les FGF, VEGF, TGF-alpha, TGF-bêta, IGF, PLGF,
où ledit épiderme sur derme est préparé de la façon suivante :
- préparation d'un échafaudage imprimé en 3D avec nanofibres pour la simulation d'un épiderme et d'un derme, où :
- l'échafaudage est imprimé en 3D en utilisant un matériau approprié,
- la simulation de l'ECM est effectuée soit par :
∘ l'électrofilage de nanofibres à partir d'un matériau sélectionné dans le groupe comprenant le CMC, l'alginate, l'oxyde de polyéthylène, le PCL, le PVA et des combinaisons de ces éléments, de préférence le CMC et l'oxyde de polyéthylène, pour former une ECM simulée, où les conditions appropriées pour la croissance cellulaire sont assurées, où le matériau polymérique de base peut être complété par un matériau supplémentaire sélectionné dans le groupe comprenant les matériaux qui forment l'ECM dans la peau, par exemple les protéoglycanes, l'acide hyaluronique, le collagène, la fibronectine), soit
∘ l'ajout de cellulose nanofibrillaire (NFC), qui assure également la morphologie de l'ECM et un environnement approprié pour stimuler la prolifération des cellules cutanées,
- l'ajout de fibroblastes et d'un facteur de croissance sélectionné dans le groupe comprenant les FGF, VEGF, TGF-alpha, TGF-bêta, IGF, PLGF, pour former le derme, où les fibroblastes et les facteurs de croissance sont ajoutés en même temps que les matériaux pour la simulation de l'ECM ou séparément,
- l'ajout de kératinocytes et d'un facteur de croissance sélectionné dans le groupe comprenant les EGF, FGF, PDGF, idéalement l'EGF, au derme formé, afin de constituer une couche d'épiderme sur le derme déjà formé,
- de préférence, la répétition des étapes i à iii, pour former une deuxième couche de derme séparée, conçue pour permettre l'impression de canaux creux,
- une couche hypodermique comprenant :
∘ du tissu sous-cutané avec adipocytes, de préférence des adipocytes somatiques, et
∘ des structures creuses avec cellules endothéliales pour la simulation de vaisseaux sanguins, où la circulation dans lesdites structures creuses est permise par au moins une micro-pompe simulant la circulation et la pression sanguines à l'intérieur des structures creuses, où l'intérieur des structures creuses est pourvu d'au moins un capteur conçu pour mesurer la circulation, la pression, la température et/ou la saturation, et où lesdites structures creuses sont préparées en utilisant l'impression 3D, de préférence l'impression 3D avec noyau/enveloppe et des cellules,
où la couche hypodermique est préparée de la façon suivante :
- structures creuses imprimées avec une impression 3D avec noyau-enveloppe sur la base d'un fichier d'impression approprié préparé préalablement, qui définit la géométrie et la distribution des structures creuses,
- ajout de cellules endothéliales, soit dans la solution destinée à l'impression des structures creuses, soit séparément, de façon à ce qu'elles puissent adhérer à la formation déjà préparée des structures creuses,
- installation de capteurs, en particulier de capteurs pour la circulation, la pression, la température, la saturation, dans les structures creuses préparées,
- ajout de fibroblastes pour une liaison améliorée entre les couches préparées aux étapes a) et b) et/ou pour la réalisation de ladite étape v), pour former un derme préparé de façon appropriée, doté de structures creuses simulant le système vasculaire,
et où sous la couche hypodermique, une couche de simulation d'os est pourvue, ladite couche de simulation d'os comprenant un substrat métallique et une couche de polysaccharides comprenant une combinaison d'alginate, de méthylcellulose et de cellulose nanofibrillaire comprenant de l'hydroxyapatite, appliquée sur ledit substrat métallique, où des ostéoblastes humains sont cultivés sur ladite couche de polysaccharides.

2. Modèle complexe *in vitro* de peau humaine selon la revendication 1, dans lequel des adipocytes sont isolés à partir de tissu adipeux humain.

3. Modèle complexe *in vitro* de peau humaine selon l'une quelconque des revendications précédentes, dans lequel l'épaisseur des couches individuelles du modèle *in vitro* de peau est arbitraire, et dans lequel les épaisseurs privilégiées sont : pour l'épiderme, de 80 à 200 µm, pour le derme, de 1500 à 5000 µm, pour l'hypoderme (tissu sous-cutané), de 800 à 2500 µm.

4. Modèle complexe *in vitro* de peau humaine selon l'une quelconque des revendications précédentes, dans lequel le modèle de peau est préparé avec tout procédé d'impression 3D approprié, à savoir par extrusion, laser, par procédé photolithographique ou par gouttelettes, de préférence avec une impression 3D par extrusion.

5. Modèle complexe *in vitro* de peau humaine selon l'une quelconque des revendications précédentes, dans lequel la simulation de matrice extracellulaire est réalisée avec des matériaux de support polymériques biocompatibles électrofilés avec cellules intégrées, ou ajout de cellulose nanofibrillaire (NFC).

6. Modèle complexe *in vitro* de peau humaine selon l'une quelconque des revendications précédentes, dans lequel ledit polysaccharide pour la simulation de matrice extracellulaire est sélectionné dans un groupe comprenant :
- des matériaux naturels tels que :
∘ les dérivés de polysaccharides : alginate (ALG), carboxyméthylcellulose (CMC), cellulose nanofibrillaire (NFC), méthylcellulose (MC),
∘viscose,
∘ soie,
∘ collagène, élastine, protéoglycanes, agrécanes, glycosaminoglycanes (acide hyaluronique), héparine, fibrinogène,
∘ toute combinaison de ces éléments,
- matériaux semi-synthétiques tels que
∘ chitosane avec dérivés,
∘ polycaprolactone,
∘ toute combinaison de ces éléments,
- matériaux synthétiques tels que :
∘ polytéréphtalate d'éthylène (PET),
∘ polytéréphtalate de butylène (PBT),
∘ polypropylène (PP),
∘ polyméthacrylate d'hydroxyéthyle,
∘ polyméthacrylate d'hydroxybutyle,
∘ alcool polyvinylique,
∘ oxyde de polyéthylène, et
∘ toute combinaison de ces éléments,
- toute combinaison de matériaux naturels, semi-synthétiques et synthétiques.

7. Modèle complexe *in vitro* de peau humaine selon l'une quelconque des revendications précédentes, dans lequel pour la simulation de matrice extracellulaire et la simulation d'os, une combinaison de polysaccharides comprenant alginate, méthylcellulose, cellulose nanofibrillaire (NFC) avec hydroxyapatite est utilisée, pour laquelle les quantités privilégiées sont :
- 10 % de méthylcellulose,
- 5 % d'alginate,
- 1,5 % de NFC et
- 1 à 5 % de CaCl₂ ou 5 % de SrCl₂.

8. Modèle complexe *in vitro* de peau humaine selon l'une quelconque des revendications précédentes, dans lequel l'irrigation est effectuée avec des pompes ou micro-pompes péristaltiques ayant un débit maximal de 100 ml par minute et un volume allant jusqu'à 10 dm³, où lesdites pompes sont contrôlées en utilisant une modulation de largeur d'impulsion.

9. Modèle complexe *in vitro* de peau humaine selon l'une quelconque des revendications précédentes, dans lequel la structure creuse est raccordée à un tube externe constitué d'un matériau approprié tel que le PP, le PET, qui peut être raccordé à la pompe au moyen d'une attelle vasculaire, où ledit tube n'est pas en contact avec les cellules du modèle *in vitro,* mais doit être non-toxique et stérile, où ladite attelle vasculaire fait office de connexion et un joint d'étanchéité entre la structure creuse et le tube empêche toute contamination des cellules par des éléments extérieurs.

10. Un procédé de préparation du modèle complexe *in vitro* de peau humaine selon l'une quelconque des revendications précédentes, **caractérisé par le fait que** le procédé comprend les étapes suivantes :
a) la préparation d'un échafaudage imprimé en 3D avec nanofibres pour la simulation d'un épiderme et d'un derme, où :
i. l'armature est imprimée en 3D en utilisant un matériau approprié,
ii. la simulation de l'ECM est effectuée soit par :
l'électrofilage de nanofibres à partir d'un matériau sélectionné dans le groupe comprenant le CMC, l'alginate, l'oxyde de polyéthylène, le PCL, le PVA et des combinaisons de ces éléments, de préférence le CMC et l'oxyde de polyéthylène, pour former une ECM simulée, où les conditions appropriées pour la croissance cellulaire sont assurées, où le matériau polymérique de base peut être complété par un matériau supplémentaire sélectionné dans le groupe comprenant les matériaux qui forment l'ECM dans la peau, par exemple les protéoglycanes, l'acide hyaluronique, le collagène, la fibronectine), soit
l'ajout de cellulose nanofibrillaire (NFC), qui assure également la morphologie de l'ECM et un environnement approprié pour stimuler la prolifération des cellules cutanées,
iii. l'ajout de fibroblastes et d'un facteur de croissance sélectionné dans un groupe comprenant les FGF, VEGF, TGF-alpha, TGF-bêta, IGF, PLGF, pour former le derme, où les fibroblastes et les facteurs de croissance sont ajoutés en même temps que les matériaux pour la simulation de l'ECM ou séparément,
iv. l'ajout de kératinocytes et d'un facteur de croissance sélectionné dans un groupe comprenant l'EGF, le FGF, le PDGF, au derme formé, afin de constituer une couche d'épiderme sur le derme déjà formé,
v. de préférence, la répétition des étapes i à iii, pour former une deuxième couche de derme séparée, conçue pour permettre l'impression de canaux creux,
b) la préparation d'un système circulatoire imprimé en 3D pour la simulation de la circulation sanguine, dans lequel :
i. les structures creuses sont imprimées avec une impression 3D avec noyau-enveloppe, sur la base d'un fichier d'impression approprié préparé préalablement, qui définit la géométrie et la distribution des structures creuses,
ii. l'ajout de cellules endothéliales, soit dans la solution destinée à l'impression des structures creuses, soit séparément, de façon à ce qu'elles puissent adhérer à la formation déjà préparée des structures creuses,
iii. l'installation de capteurs, en particulier de capteurs pour la circulation, la pression, la température, la saturation, dans les structures creuses préparées,
iv. l'ajout de fibroblastes pour une liaison améliorée entre les couches préparées aux étapes a) et b) et/ou pour la réalisation de ladite étape v), pour former un derme préparé de façon appropriée et doté de structures creuses simulant le système vasculaire,
c) la préparation de tissu sous-cutané, dans laquelle une combinaison de matériaux appropriés est utilisée pour l'impression en 3D, de préférence de l'ALG, du MC dans la NFC, avec des adipocytes,
d) facultativement, le raccordement d'au moins une pompe appropriée pour assurer une circulation dans le système vasculaire simulé, où la connexion est assurée par une attelle vasculaire en Teflon installée sur un tube en silicone biocompatible qui permet le fonctionnement à des pressions appropriées et la circulation des nutriments dans le système vasculaire, et où des connexions alternatives au moyen d'autres matériaux biocompatibles sont aussi possibles,
e) la préparation d'un substrat métallique et impression en 3D d'une couche de polysaccharides comprenant une combinaison d'alginate, de méthylcellulose et de cellulose nanofibrillaire avec de l'hydroxyapatite, comprenant des ostéoblastes humains *in situ,*
f) l'incubation du modèle de peau *in vitro* obtenu dans un endroit approprié et dans des conditions adéquates et facultativement, prolifération supplémentaire du tissu préparé.

11. Procédé selon la revendication 14 ou 15, **caractérisé par le fait qu'**il comprend les étapes suivantes :
- les matériaux ALG, MC, NFC, les fibroblastes et le facteur de croissance FGF sont mélangés et imprimés avec une imprimante 3D pour obtenir une structure de derme en 3D,
- sur le derme obtenu, des kératinocytes avec le facteur de croissance EGF sont pourvus et incubés pour qu'ils prolifèrent pendant 3 jours, pour permettre la formation d'un épiderme sur la couche de derme,
- les matériaux ALG, MC, NFC et les cellules endothéliales sont imprimés sous la forme de tubes pour simuler le système vasculaire, autour desdits tubes, une combinaison de matériaux ALG, MC, NFC, des fibroblastes et le facteur de croissance FGF sont imprimés, pour obtenir des vaisseaux sanguins simulés dans la partie la plus basse du derme,
- les matériaux ALG, MC, NFC et les adipocytes sont imprimés avec une imprimante 3D sous la structure dotée de tubes creux, afin de former une couche de tissu sous-cutané, et
- le matériau support est constitué d'acier inoxydable de qualité médicale.

12. Utilisation *in vitro* du modèle complexe *in vitro* de peau humaine selon l'une quelconque des revendications 1 à 9 en médecine, en pharmacie, en biotechnologie et dans les tests de matériaux, de produits chimiques, substances pharmaceutiques, produits cosmétiques et composés similaires, de préférence dans :
- le développement et les tests de médicaments novateurs pour la peau, par exemple les médicaments anti-inflammatoires pour la peau, les antibiotiques locaux, les substances désinfectantes, et/ou
- le développement et les tests de produits transdermiques, tels que les patchs transdermiques pour soulager la douleur, pour hormones, etc., et/ou
- les tests *in vitro* de préparations transdermiques pharmaceutiques et cosmétiques, par exemple mesure de la concentration d'un principe actif lors du passage à travers la surface de la peau dans le système vasculaire, et/ou
- l'étude du transfert de principes actifs du système vasculaire dans le tissu environnant, et/ou
- la possibilité de mesurer des paramètres tels que le glucose, l'hémoglobine glyquée, la CRP et le cholestérol via la surface de la peau, et/ou
- l'étude de l'effet d'influences physiologiques, telles que la température du corps, l'activité cellulaire, etc., sur le comportement de composés ou préparations actifs testés, et/ou
- les tests de toxicité sur les cellules de la peau et évaluation de la biocompatibilité des préparations testées, et/ou
- le développement et les tests de capteur non-invasif pour la mesure du glucose ou d'autres capteurs non-invasifs, mesure des propriétés à travers ou sur la peau.
